# EUROPEAN PATENT SPECIFICATION

(11) **EP 1 382 358 B1**
(45) Date of publication and mention of the grant of the patent: **06.08.2008**
(21) Application number: 03015535.2
(22) Date of filing: 10.07.2003
(51) Int. Cl.: A61M 1/16, A61M 1/10, A61M 1/36

(54) **Device for treating blood in an extracorporeal circuit**
Vorrichtung zur Blutbehandlung in einem extrakorporalen Kreislauf
Dispositif pour le traitement du sang dans un circuit extracorporel

(30) Priority: 15.07.2002 IT MI20021552
(43) Date of publication of application: 21.01.2004
(73) Proprietor: Sorin Group Italia S.r.l., 20159 Milano (IT)
(72) Inventor: Costa Maianti, Edgardo, 41037 Mirandola (Modena) (IT); Ghelli, Nicola, 40018 San Pietro in Casale (BO) (IT); Panzani, Ivo, 41037 Mirandola (Modena) (IT)
(74) Representative: Modiano, Micaela Nadia

(56) References cited:
- EP-A- 0 724 889
- EP-A- 0 987 035
- EP-A- 1 180 374
- US-A- 4 490 331
- US-A- 5 770 149

## Description

The present invention relates to a device for treating blood in an extracorporeal circuit.

It is known that during certain surgical procedures it is necessary to establish an extracorporeal circulation of the blood of the patient in a circuit that comprises devices suitable to ensure correct treatment of blood. Such devices comprise at least one reservoir for containing the blood, termed venous blood, that leaves the patient, a pump for conveying the blood in the circuit, a heat exchanger in which the blood encounters a heat exchange fluid that ensures its correct temperature, an oxygenation apparatus meant to transfer oxygen to the blood, and finally a filter that is interposed on the line, known as arterial blood line, that returns the blood to the patient, with the purpose of retaining any air bubbles that are present in the blood. The described extracorporeal circuit is completed by the presence of a container for the blood collected by salvage from the operating field, known as a cardiotomy reservoir, which is connected to the venous blood reservoir.

Originally, extracorporeal circuits comprised the described devices as independent elements that were interconnected by virtue of connecting lines. In order to improve the functional parameters of the circuit, particularly minimizing hemodilution, hemolysis and risk of embolisms, and in order to improve oxygen transport and streamline the distribution of the components around the operating field, partial integrations of the devices have been made in the prior art.

The aim of the present invention is now to provide a device for treating blood in an extracorporeal circuit that allows minimizing the filling volume, i.e., the volume of the blood that is present outside the body of the patient, the surface in contact with the blood, and the overall dimensions, so as to ensure an optimum treatment of the blood in the circuit and convenient management on the part of operators. This aim can be achieved by a device for treating blood in an extracorporeal circuit, that comprises, integrated in a single monolithic assembly, a venous blood reservoir that is connected to a line for conveying the blood that arrives from the patient, a heat exchanger, a pump, an oxygenation apparatus and an arterial blood filter connected to a line for returning the blood to the patient.

The invention provides a device for treating blood in an extracorporeal circuit as defined in claim 1.

Additional features and advantages of the invention are set forth in the description which follows and in part will be apparent from the description. The objectives and other advantages of the invention will be realized and attained by the device for treating blood in an extracorporeal circuit particularly pointed out in the written description and claims.

It is to be understood that both the foregoing general description and the following detailed description are exemplary and explanatory and are intended to provide further explanation of the invention as claimed.

Further characteristics and advantages will become apparent from the description of two preferred but not exclusive embodiments of the invention which are illustrated by way of non-limitative example in the accompanying drawings.

Figure 1 is a diagram of a prior art extracorporeal circuit.

Figures 2 and 4 are diagrams of different embodiments of an extracorporeal circuit that comprises the device for treating blood.

Figures 3 and 5 are cross-sectional views of the devices illustrated in Figures 2 and 4, respectively.

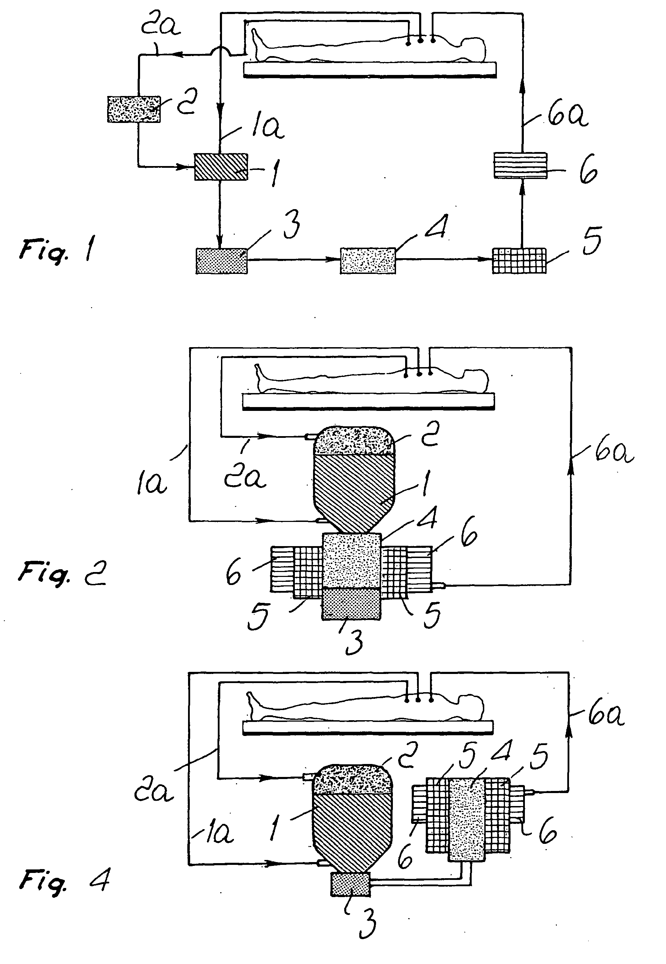

With reference to Figure 1, the reference numeral 1a designates the venous blood line that conveys the blood from the patient P to the venous blood reservoir 1, which also receives blood from the cardiotomy reservoir 2 for collection from the operating field (i.e., from patient P) by means of the line 2a. The reference numeral 3 designates the pump, reference numeral 4 designates the heat exchanger, and reference numeral 5 designates the oxygenation apparatus, from which arterial blood line 6a leads out, with arterial blood filter 6 interposed, returning the blood to the patient P.

As mentioned above, originally extracorporeal circuits comprised all the devices as independent elements connected by connecting lines. According to this invention, the described devices are integrated into a single device and are designated, in the subsequent figures, by the same reference numerals adopted in Figure 1. Furthermore, in Figures 2 and 4, the individual devices are shown with the same graphical identification as shown in Figure 1.

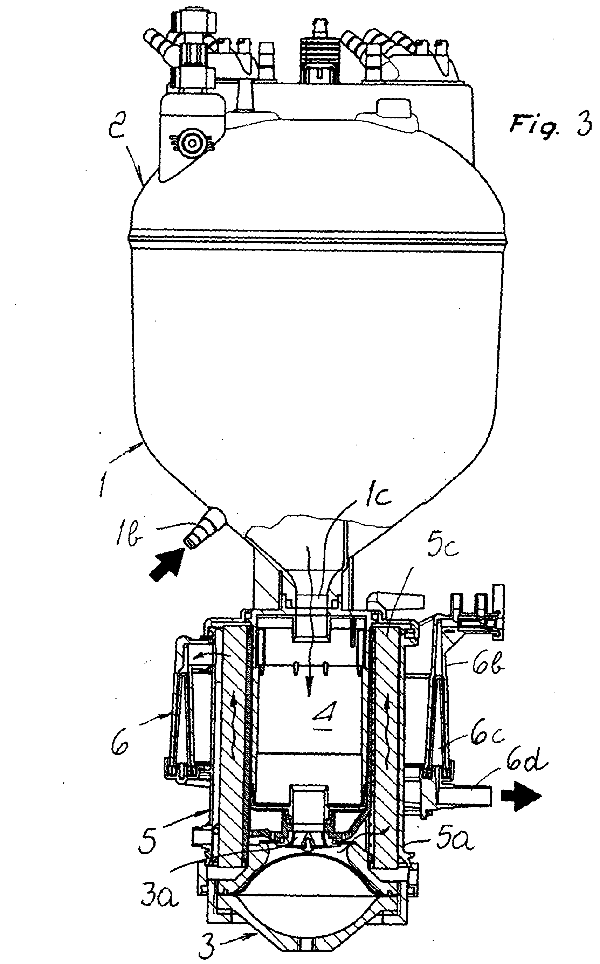

A device for treating blood is described with reference to Figures 2 and 3. In this device, oxygenation apparatus 5 comprises hollow cylindrical structure 5 a for containing blood oxygenation structures 5b such as hollow fibers and is suitable to contain the heat exchanger 4. Structure 5a supports, at its upper face, venous blood reservoir 1, which is connected to the patient P by means of venous blood line 1 a that reaches connector 1b, and supports, at its lower face, pump 3, which is of the pulsating type. There is also annular structure 6b of arterial blood filter 6, which is monolithically connected to structure 5a and is suitable to contain filtration structure 6c for the arterial blood that returns to the patient P by means of arterial blood line 6a that leads out from connector 6d. Cardiotomy reservoir 2 is integrated in a position above the venous blood reservoir 1 and is connected to the operating field by the line 2a. The venous blood reservoir 1 lid comprises a passage port that is provided with an occlusion device.

In the described device, the blood follows the arrows shown in Figure 3, and therefore, once it has entered venous blood reservoir 1 from connector 1b, it is introduced by means of output connector 1 c of the reservoir into heat exchanger 4, which has a coaxial and directly facing inlet connector. At the outlet of heat exchanger 4, the blood enters the coaxial and directly facing intake duct 3a of pump 3, is sent by the pump to the inlet of oxygenation apparatus 5, and then exits the apparatus and enters arterial blood filter 6 so as to be able to return to the patient P by means of arterial blood line 6a connected to connector 6d.

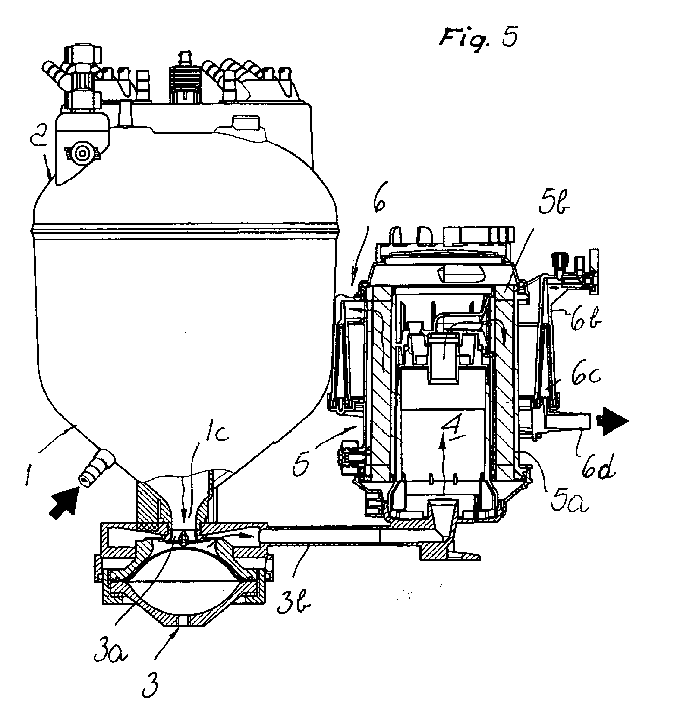

A device for treating blood according to the invention is now described with reference to Figures 4 and 5. In this embodiment, intake duct 3a of pump 3 is connected directly to output connector 1c of venous blood reservoir 1, which receives the blood from venous blood line 1a connected to inlet connector 1b and supports cardiotomy reservoir 2, which is integrated therein in an upward region. Delivery duct 3b of pump 3 ends at the base of hollow cylindrical structure 5a of oxygenation apparatus 5, which contains blood oxygenation structures 5b such as hollow fibers for blood oxygenation, and is suitable to accommodate heat exchanger 4. Annular structure 6b of arterial blood filter 6 is provided at the peripheral region of structure 5a and is monolithically connected thereto. The structure 6b is suitable to contain filtration structure 6c for filtering the arterial blood that returns to the patient P by means of arterial blood line 6a connected to connector 6d. As shown in Figure 5, annular structure 6b is monolithically connected to venous blood reservoir 1 at portion 7.

The arrows of Figure 5 illustrate the path of the blood, which is introduced in pump 3 from venous blood reservoir 1 and is conveyed by delivery tube 3b of the pump so that it arrives at the base of structure 5a at the axis thereof, thus facing directly the inlet of heat exchanger 4. When the blood leaves exchanger 4, it enters oxygenation apparatus 5 and then passes into arterial blood filter 6, subsequently returning to the patient P by means of arterial blood line 6a connected to connector 6d.

The above description and accompanying drawings 4 and 5 are provided for the purpose of describing an embodiment of the invention and are not intended to limit the scope of the invention in any way. It will be apparent to those skilled in the art that various modifications and variations can be made in the device for treating blood in an extracorporeal circuit without departing from the scope of the invention. For example, the pulsating pump can be replaced with a centrifugal pump. Thus, it is intended that the present invention cover the modifications and variations of this invention provided they come within the scope of the appended claims and their equivalents.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device for treating blood in an extracorporeal circuit comprising a venous blood reservoir (1) having an inlet and an outlet, a heat exchanger (4) having an inlet and an outlet, a pump (3) having an inlet and an outlet, an oxygenation apparatus (5) having an inlet and an outlet, and an arterial blood filter (6) having an inlet and an outlet, the venous blood reservoir (1), heat exchanger (4), pump (3), oxygenation apparatus (5), and arterial blood filter (6) being integrated into a single monolithic assembly, wherein the outlet (1c) of the venous reservoir (1) is connected to the inlet (3a) of the pump (3), the outlet (3b) of the pump (3) is connected to the inlet of the heat exchanger, the outlet of the heat exchanger is connected to the inlet of the oxygenation apparatus, the outlet of the oxygenation apparatus is connected to the inlet of the arterial filter, and the inlet (3a) of the pump (3) is connected directly to the outlet (1c) of the venous blood reservoir (1), **characterized in that** the outlet (3b) of the pump (3) ends at the base of a first hollow cylindrical structure (5a) for containing a blood oxygenation structure (5a), and **in that** the first hollow cylindrical structure is suitable to accommodate the heat exchanger (4) and to support, at the peripheral region, a second hollow cylindrical structure (5b) that is suitable to contain a filtration structure (6c) for filtering the arterial blood, and **in that** the pump (3) is placed in a position below the venous reservoir (1) and at a level lower than the oxygenation apparatus.

2. The device according to claim 1, **characterized in that** it further comprises a cardiotomy reservoir (2) that is monolithically connected to the venous blood reservoir (1).

3. The device according to claim 1, **characterized in that** the outlet (3b) of the pump (3) ends at the base of the first hollow cylindrical structure (5a) at the axis thereof and directly faces the inlet of the heat exchanger provided within the first hollow cylindrical structure (5a).

4. The device according to one or more of the preceding claims, **characterized in that** the pump (3) is a pulsating pump.

5. The device according to one or more of the preceding claims, **characterized in that** the pump (3) is a centrifugal pump.

## Patentansprüche

1. Eine Vorrichtung zur Behandlung von Blut in einem Extrakorporalkreislauf, die Folgendes umfasst: einen Venenblut-Behälter (1) mit einem Einlass und einem Auslass, einen Wärmetauscher (4) mit einem Einlass und einem Auslass, eine Pumpe (3) mit einem Einlass und einem Auslass, eine Oxygenierungs-Vorrichtung (5) mit einem Einlass und einem Auslass und einen Arterienblut-Filter (6) mit einem Einlass und einem Auslass, wobei der Venenblut-Behälter (1), der Wärmetauscher (4), die Pumpe (3), die Oxygenierungs-Vorrichtung (5) und der Arterienblut-Filter (6) in einen einzigen monolithischen Aufbau integriert sind, wobei der Auslass (1c) des Venenblut-Behälters (1) mit dem Einlass (3a) der Pumpe (3) verbunden ist, der Auslass (3b) der Pumpe (3) mit dem Einlass des Wärmetauschers verbunden ist, der Auslass des Wärmetauschers mit dem Einlass der Oxygenierungs-Vorrichtung verbunden ist, der Auslass der Oxygenierungs-Vorrichtung mit dem Einlass des Arterienblut-Filters verbunden ist, und wobei der Einlass (3a) der Pumpe (3) direkt mit dem Auslass (1c) des Venenblut-Behälters (1) verbunden ist, **dadurch gekennzeichnet, dass** der Auslass (3b) der Pumpe (3) an der Basis einer ersten hohlen zylindrischen Struktur (5a) zum Enthalten einer Blut-Oxygenierungs-Struktur (5a) endet, und **dadurch**, dass die erste hohle zylindrische Struktur geeignet ist, den Wärmetauscher (4) aufzunehmen und im peripheren Bereich eine zweite hohle zylindrische Struktur (5b) zu tragen, die geeignet ist, eine Filtrationsstruktur (6c) zum Filtrieren des Arterienbluts zu enthalten, und **dadurch**, dass die Pumpe (3) in eine Position unterhalb des Venenblut-Behälters (1) und in einer Höhe platziert wird, die unterhalb der Oxygenierungs-Vorrichtung liegt.

2. Die Vorrichtung gemäß Anspruch 1, **dadurch gekennzeichnet, dass** sie weiter einen Kardiotomie-Behälter (2) umfasst, der monolithisch mit dem Venenblut-Behälter (1) verbunden ist.

3. Die Vorrichtung gemäß Anspruch 1, **dadurch gekennzeichnet, dass** der Auslass (3b) der Pumpe (3) an der Basis der ersten hohlen zylindrischen Struktur (5a) an der Achse davon endet und direkt dem Einlass des Wärmetauschers gegenüberliegt, der in der ersten hohlen zylindrischen Struktur (5a) bereitgestellt wird.

4. Die Vorrichtung gemäß einem oder mehreren der obigen Anspruche, **dadurch gekennzeichnet, dass** die Pumpe (3) eine pulsierende Pumpe ist.

5. Die Vorrichtung gemäß einem oder mehreren der obigen Ansprüche, **dadurch gekennzeichnet, dass** die Pumpe (3) eine Zentrifugalpumpe ist.

## Revendications

1. Dispositif de traitement du sang dans un circuit extracorporel comprenant un réservoir de sang veineux (1) ayant une entrée et une sortie, un échangeur de chaleur (4) ayant une entrée et une sortie, une pompe (3) ayant une entrée et une sortie, un appareil d'oxygénation (5) ayant une entrée et une sortie, et un filtre de sang artériel (6) ayant une entrée et une sortie, le réservoir de sang veineux (1), l'échangeur de chaleur (4), la pompe (3), l'appareil d'oxygénation (5) et le filtre de sang artériel (6) étant intégrés en un ensemble monolithique unique, dans lequel la sortie (1c) du réservoir de sang veineux (1) est reliée à l'entrée (3a) de la pompe (3), la sortie (3b) de la pompe (3) est reliée à l'entrée de l'échangeur de chaleur, la sortie de l'échangeur de chaleur est reliée à l'entrée de l'appareil d'oxygénation, la sortie de l'appareil d'oxygénation est reliée à l'entrée du filtre de sang artériel et l'entrée (3a) de la pompe (3) est reliée directement à la sortie (1c) du réservoir de sang veineux (1), **caractérisé en ce que** la sortie (3b) de la pompe (3) aboutit à la base d'une première structure cylindrique creuse (5a) pour contenir une structure d'oxygénation du sang (Sa), et **en ce que** la première structure cylindrique creuse est adaptée à recevoir l'échangeur de chaleur (4) et à supporter, au niveau de la région périphérique, une seconde structure cylindrique creuse (5b) qui est adaptée à contenir une structure de filtration (6c) pour filtrer le sang artériel, et **en ce que** la pompe (3) est placée dans une position au-dessous du réservoir de sang veineux (1) et à un niveau inférieur à l'appareil d'oxygénation.

2. Dispositif selon la revendication 1, **caractérisé en ce qu'**il comprend de plus un réservoir de cardiotomie (2) qui est relié de façon monolithique au réservoir de sang veineux (1).

3. Dispositif selon la revendication 1, **caractérisé en ce que** la sortie (3b) de la pompe (3) aboutit à la base de la première structure cylindrique creuse (5a) au niveau de son axe et fait face directement à l'entrée de l'échangeur de chaleur prévu à l'intérieur de la première structure cylindrique creuse (5a).

4. Dispositif selon une ou plusieurs des revendications précédentes, **caractérisé en ce que** la pompe (3) est une pompe à impulsions.

5. Dispositif selon une ou plusieurs des revendications précédentes, **caractérisé en ce que** la pompe (3) est une pompe centrifuge.
